(19) Europäisches Patentamt
European Patent Office
Office européen des brevets

(11) Publication number: **0 065 364**
**B1**

(12) **EUROPEAN PATENT SPECIFICATION**

(45) Date of publication of patent specification: **27.12.84**

(51) Int. Cl.$^3$: **C 07 C 15/067,** C 07 C 5/27

(21) Application number: **82302140.7**

(22) Date of filing: **26.04.82**

(54) **Process for the isomerization of alkylbenzenes.**

(30) Priority: **20.05.81 GB 8115411**

(43) Date of publication of application:
**24.11.82 Bulletin 82/47**

(45) Publication of the grant of the patent:
**27.12.84 Bulletin 84/52**

(84) Designated Contracting States:
**BE DE FR GB IT NL**

(56) References cited:
**EP-A-0 018 090**
**EP-A-0 021 445**
**US-A-3 856 871**
**US-A-3 856 872**
**US-A-4 152 363**
**US-A-4 268 420**

(73) Proprietor: **IMPERIAL CHEMICAL INDUSTRIES PLC**
**Imperial Chemical House Millbank**
**London SW1P 3JF (GB)**

(72) Inventor: **Lake, Ivan James Samuel**
**75 Clevegate Runnymede Park Nunthorpe**
**Middlesbrough Cleveland (GB)**
Inventor: **Whittam, Thomas Vincent**
**30 Wilton Drive**
**Darlington Durham (GB)**

(74) Representative: **Martin, David Lincoln et al**
**Imperial Chemical Industries PLC Legal**
**Department: Patents PO Box 6 Bessemer Road**
**Welwyn Garden City Herts, AL7 1HD (GB)**

**EP 0 065 364 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a process for the isomerisation of alkyl benzene hydrocarbons using a catalyst comprising a new zeolite material. The new zeolite material will be referred to hereinafter as "zeolite Nu4" or simply "Nu4".

The isomerisation of alkylbenzene hydrocarbons is a wellknown process and a number of catalysts have been proposed for use in the process. U.S. Patent No. 3,856,871 describes the isomerisation of xylenes in which the $C_8$ aromatics mixture is contacted with a catalyst which comprises an acid zeolite of the ZSM-5, ZSM-11, or ZSM-21 type in the liquid phase and in the absence of added hydrogen at a temperature of 500 to 660°F. U.S. Patent No. 4,268,420 proposes the use in the xylenes isomerisation process of a catalyst comprising a crystalline borosilicate and a porous refractory oxide.

Zeolite Nu4 and its preparation are described in our copending European Patent Application published as EPA No. 65401A.

Zeolite Nu4 has a chemical composition, in terms of mole ratios of oxides, expressed by the formula

$$0.1 \text{ to } 7.5\ M'_2O : 0.2 \text{ to } 5\ Y_2O_3 : 100\ XO_2 : 0 \text{ to } 50\ H_2O$$

wherein M' is a monovalent cation or 1/n of a cation of valency n, X is silicon or germanium, Y is aluminium, iron, chromium, vanadium, molybdenum, arsenic, antimony, manganese, gallium, or boron, and $H_2O$ is water of hydration additional to water notionally present when M' is H, and has an X-ray pattern substantially as set out in Table 1 (as determined by standard technique using Copper K$\alpha$ radiation). Table 1 shows X-ray data for zeolite Nu4 as prepared with reference to the Examples in our co-pending European Patent Application No. 65401A. The X-ray pattern is little affected by the type of cation present or by calcination or hydration.

TABLE 1

| Zeolite Nu4 Example 3 (as made) | | Zeolite Nu4 Example 4 (calcined H form) | |
|---|---|---|---|
| dA | 100I/Io | dA | 100I/Io |
| 11.3 | 16 | 11.07 | 33 |
| 11.1 | 20 | 10.07 | 36 |
| 10.08 | 15 | 9.96 | 10 |
| 9.90 | 8 | 9.79 | 10 |
| 9.77 | 6 | 9.28 | 1 |
| 9.05 | 1 | 9.02 | 1.5 |
| 7.50 | 2 | 8.08 | 1 |
| 7.09 | 1 | 7.45 | 4 |
| 6.78 | 2 | 7.09 | 2 |
| 6.44 | 5 | 6.72 | 4 |
| 6.07 | 4 | 6.38 | 8 |
| 6.05 | 5 | 6.08 | 5 |
| 5.97 | 1 | 6.02 | 7 |
| 5.75 | 8 | 5.99 | 7 |
| 5.65 | 6 | 5.73 | 9 |
| 5.63 | 2 | 5.57 | 8 |

TABLE 1 (contd)

| Zeolite Nu4 Example 3 (as made) dA | 100I/Io | Zeolite Nu4 Example 4 (calcined H form) dA | 100I/Io |
|---|---|---|---|
| 5.41 | 2 | 5.38 | 2 |
| 5.19 | 2 | 5.15 | 4 |
| 5.07 | 2 | 5.04 | 3 |
| 5.01 | 4 | 5.005 | 5 |
| 4.915 | 1 | 4.899 | 1 |
| 4.629 | 9 | 4.725 | 1 |
| 4.558 | 1 | 4.610 | 9 |
| 4.495 | 2 | 4.560 | 2 |
| 4.475 | 2 | 4.490 | 1 |
| 4.386 | 13 | 4.470 | 2 |
| 4.291 | 10 | 4.380 | 14 |
| 4.124 | 13 | 4.280 | 13 |
| 4.104 | 4 | 4.103 | 3 |
| 4.039 | 6 | 4.022 | 7 |
| 3.950 | 1 | 3.950 | 2 |
| 3.880 | 100 | 3.869 | 100 |
| 3.860 | 69 | 3.836 | 73 |
| 3.743 | 51 | 3.764 | 35 |
| 3.730 | 50 | 3.735 | 54 |
| 3.678 | 27 | 3.662 | 29 |
| 3.649 | 22 | 3.604 | 4 |
| 3.629 | 5 | 3.500 | 5 |
| 3.466 | 12 | 3.469 | 10 |
| 3.364 | 6 | 3.368 | 7 |
| 3.332 | 9 | 3.342 | 10 |
| 3.329 | 4 | 3.265 | 3 |
| 3.273 | 4 | 3.260 | 12 |
| 3.267 | 4 | | |
| 3.260 | 4 | | |

Within the above definition of chemical composition, the number of moles of $Y_2O_3$ is typically in the range 0.2 to 5 and zeolite Nu4 appears to be most readily formed in a state of high purity when the number of moles of $Y_2O_3$ is in the range 0.5 to 2.

This definition includes both freshly prepared zeolite Nu4 ("freshly prepared" means the product of synthesis and washing, with optional drying, as described in our co-pending European Patent Application No. 65401A) and also forms of it resulting from dehydration, and/or calcination, and/or ion exchange. In freshly prepared zeolite Nu4, M' may include an alkali metal cation especially sodium, and/or ammonium, and usually or when prepared from nitrogen compounds, includes nitrogen-containing organic cations as described below or cationic degradation products thereof, or precursors thereof. These nitrogen containing cations are hereinafter referred to as Q. The alkali metal cation comprising M' may also be potassium or rubidium but the Applicants have found that use of these two cations during synthesis of zeolite Nu-4 may also result in the co-production of zeolite Nu-10 as an impurity (Zeolite Nu-10 is described and claimed in our co-pending European Patent Application No. 82302366.8 published as EPA No. 65400A).

The freshly prepared zeolite Nu4 may also contain nitrogen-containing compounds typically in the range 0.4 to 6 moles per mole of $XO_2$. Since Nu4 is a zeolite, the nitrogen-containing base must be physically trapped within the crystal lattice. It can be removed by thermal or oxidative degradation or by displacement by suitable small molecules. This physically trapped basic material does not constitute part of the composition for the purposes of the definition. Thus a zeolite Nu-4 as made typically has the following molar composition:

$$0.1 \text{ to } 7.5\ M'_2O : 0.4 \text{ to } 6\ Q : 0.2 \text{ to } 5\ Y_2O_3 : 100\ XO_2 : 0 \text{ to } 50\ H_2O$$

wherein M' is an alkali metal or ammonium or hydrogen.

The $H_2O$ content of freshly prepared zeolite Nu4 depends on the conditions in which it has been dried after synthesis.

In calcined forms of zeolite Nu4, M' may be alkali metal but includes less or no nitrogen-containing organic compounds, since these are burnt out in the presence of air, leaving hydrogen as the other balancing cation, or otherwise displaced prior to calcination.

Among the ion-exchanged forms of zeolite Nu4 the ammonium ($NH_4^+$) is of importance since it can be readily converted to the hydrogen form by calcination. The hydrogen form can also be prepared directly by exchange with an acid. The hydrogen-free and forms containing metals introduced by ion exchange are described further below.

We believe that zeolite Nu4 is a new member of the ZSM5/11 series of zeolite, which has the lowest symmetry possible for members of this series. ZSM11 has the highest i.e. has tetragonal symmetry. ZSM5 usually exhibits orthorhombic symmetry, but recently (E L Wu et al J Phys. Chem. *83,* 2777 (1979)) it has been claimed that ZSM5, under special circumstances, can exhibit pseudo monoclinic symmetry. Specific treatments e.g. $NH_4^+$ exchange, calcination, sorption, can cause displacive transformation from orthorhombic to pseudo monoclinic symmetry. These transformations are completely reversible and in ZSM5 depend only upon the ions or sorbate present within the ZSM5 lattice.

We have found that zeolite Nu4 as made, or in all its ion-exchanged, calcined, hydrated, dehydrated or sorbing forms, always exhibits true monoclinic symmetry. This is best demonstrated by reference to Nature *275,* 120 (1978), where it is noted that the major clue to differences in structure, between the closely related zeolites ZSM5 and ZSM11, came from differences in X-ray diffraction data. In the diffraction data for ZSM5 (orthorhombic) there is a significant splitting of certain ZSM11 (tetragonal) type peaks into doublets and there are variations in peak heights. This behaviour confirms that ZSM5 has a lower order of symmetry than ZSM11, but is nevertheless closely related. In going from tetragonal ZSM5 to pseudo-monoclinic ZSM5 there is a further marginal peak splitting. However in the case of zeolite Nu4 there is very substantial splitting of peaks as compared with tetragonal or even pseudo-monoclinic ZSM5. This splitting includes many new doublets and even triplets, and also marked variations in line intensities.

According to the present invention a process for the isomerisation of alkylbenzenes comprises contacting a feed of an alkylbenzene or a mixture of alkylbenzenes under isomerisation conditions in the vapour or liquid phase with a catalyst comprising zeolite Nu4.

The catalyst used in the process of this invention may be zeolite Nu4 *per se* but in a preferred embodiment of the process zeolite Nu4 is used in the form of a physical mixture with a suitable diluent and/or binder, for example alumina, silica or a clay. Alumina is particularly preferred as a diluent, the amount of alumina present in the catalyst being preferably in the range 5 to 95% by weight based on total catalyst weight.

In the vapour phase, suitable isomerisation conditions for the process of this invention include a temperature in the range 100 to 600°C, preferable 200 to 450°C and a pressure in the range 0.5 to 50.7 bars (0.5 to 50 atmospheres absolute), preferably 1 to 5.1 bars (1 to 5 atmospheres absolute).

In the liquid phase embodiment of the process of this invention, suitable isomerisation conditions include a temperature in the range 0 to 350°C, a pressure in the range 1 to 202 bars absolute (1 to

200 atmospheres absolute), preferably 5.07 to 7.09 bars absolute (5 to 7 atmospheres absolute), and, in a flow system, a space velocity in the range 0.1 to 100, preferably 0.5 to 30, w/w hour, the higher flow rates being used at the higher temperatures. Optionally a diluent is present, suitably one or more of those having a critical temperature higher than the isomerisation temperature being used and including toluene, trimethylbenzene, naphthenes and paraffins. Preferably, the diluent if present, amounts to 1 to 90% of the feed to the isomerisation reaction. In the above mentioned forms of the process of this invention the catalyst preferably contains no hydrogenation/dehydrogenation component.

Optionally the isomerisation reaction is conducted in the presence of hydrogen. A suitable mole ratio of hydrogen to alkybenzene lies in the range 1:1 to 30:1. If hydrogen is used, it is preferred that the catalyst should comprise a metal of Group VIII of the Periodic Table together with the zeolite. Preferably the metal of Group VIII is platinum or nickel. The amount of metal used preferably lies in the range 0.1 to 2% by weight of metal based on the total weight of catalyst. If desired, the catalyst may contain one or more additional metals, for example rhenium, suitably in the range 0.1 to 2% by weight based on the total weight of catalyst.

Preferably the alkylbenzene is a xylene, for example m-xylene for conversion to p-xylene, or a mixture of xylenes, possibly with ethylbenzene. The amount of ethylbenzene present will depend to some extent on the source of the xylene mixture but will usually lie in the range 0 to 25% by weight of the feedstock. However, we believe that the process of this invention is very suitable for conversion to paraxylene of at least some of the ethylbenzene in feedstocks containing relatively large amounts of ethylbenzene, say in the range 6 to 25% by weight of the feedstock.

The invention is illustrated by the following Example.

Example 1

A sample of zeolite Nu4 prepared as described in Example 2 of our co-pending European Patent Application No. 65401A was formed into aggregates having diameters in the size range of 425 to 1000 $\mu$m.

The sample was charged to a glass reactor and heated in a stream of air at 500°C for 16 hours. It was then cooled in a nitrogen stream to 400°C. A feedstock of the following composition (all % by weight) was then passed over the sample at atmospheric pressure and 400°C.

| | |
|---|---|
| Benzene | 0.2% |
| Toluene | 1.96% |
| Ethylbenzene | 8.78% |
| Paraxylene | 8.24% |
| Metaxylene | 50.07% |
| Orthoxylene | 25.80% |
| $C_{9+}$ aromatics | 3.72% |

Results are given in Table 2.

Example 2

Example 1 was repeated using a sample of zeolite Nu4 prepared according to Example 4 of our co-pending European Patent Application No. 65401A, and formed in aggregates having diameters in the size range of 425 to 1000 $\mu$m.

Results are given in Table 2.

Example 3

Example 2 was repeated but in this case the sample of zeolite Nu4 was mixed with gamma-alumina powder in the weight ratio (zeolite:alumina) of 1:4 and again formed into aggregates having diameters in the size range of 425 to 1000 $\mu$m.

Results are given in Table 2.

Comparative Examples 1 and 2

Example 1 was repeated but using two other catalysts for comparative purposes. In comparative Example 1 a sample of zeolite ZSM-5 was used which had been prepared according to the method described in German OLS 2,548,695 and formed into aggregates having diameters in the size range 425 to 1000 $\mu$m.

In comparative Example 2 a sample of amorphous silica-alumina catalyst in the form of 3 to 5 mm beads, containing 10% alumina, was used. In this case only, the reaction temperature was 450°C.

Results of all three Examples and the two Comparative Examples are given in Table 2.

TABLE 2

| | Example 1 | Example 2 | Example 3 | Comparative Examples 1 | Comparative Examples 2 |
|---|---|---|---|---|---|
| Weight Hourly Space Velocity | 19 | 82 | 30 | 83 | 1 |
| Para-xylene content of product (%) | 17.9 | 17.8 | 17.9 | 17.9 | 17.2 |
| Xylenes lost (%) | 2.1 | 4.35 | 1.7 | 0.6 | 5.1 |
| Ethylbenzene lost (%) | 49.1 | 46.0 | 44.1 | 18.0 | 12.8 |

The results illustrate that

1. compared with amorphous silica-alumina, the zeolite Nu4 catalyst produces more paraxylene, destroys a much higher proportion of ethylbenzene and destroys less xylenes.
2. compared with zeolite ZSM5, for the same paraxylene production zeolite Nu4 destroys rather more xylenes and much more ethylbenzene.
3. dilution of zeolite Nu4 with alumina decreases the extent of xylenes loss while still maintaining the ethylbenzene destruction.

The greater ability of zeolite Nu4 to destroy ethylbenzene is likely to be a useful asset in the future. In existing xylenes plants a feedstock which is comparatively low in ethylbenzene is usually used and destruction of ethylbenzene is fairly readily accomplished. However, in the future it is likely that xylene manufacturers will have to turn to feedstocks containing much higher amounts of ethylbenzene as the low-ethylbenzene feedstocks are expected to become much less readily available. In those circumstances, the ability of a catalyst such as zeolite Nu4 to not only effect isomerisation of the other xylenes to valuable paraxylene but also destroy the greater amounts of ethylbenzene in the feedstock will be a considerable asset.

## Claims

1. A process for the isomerisation of alkylbenzenes which comprises contacting a feed of an alkylbenzene or a mixture of alkybenzenes under isomerisation conditions in the vapour or liquid phase with a catalyst characterised in that the catalyst comprises zeolite Nu-4.

2. A process as claimed in claim 1 in which the catalyst comprises zeolite Nu-4 in the form of a physical mixture with a diluent and/or a binder material.

3. A process as claimed in claim 2 in which the diluent is alumina which is present in an amount of 5 to 95% by weight based on total catalyst weight.

4. A process as claimed in any one of the preceding claims in which the process is carried out in the vapour phase at a temperature in the range 100 to 600°C and at pressure in the range 0.5 to 50.7 bars (0.5 to 50 atmospheres absolute).

5. A process as claimed in any one of claims 1 to 3 in which the process is carried out in the liquid phase at a temperature in the range 0 to 350°C and at a pressure in the range 1 to 202 bars (1 to 200 atmospheres absolute).

6. A process as claimed in any one of the preceding claims in which the process is carried out in the presence of hydrogen, the hydrogen being present in an amount such that the mole ratio of hydrogen to alkylbenzene is in the range 1:1 to 30:1.

7. A process as claimed in claim 6 in which the catalyst comprises a metal of Group VIII of the Periodic Table together with zeolite Nu-4.

8. A process as claimed in claim 7 in which the Group VIII metal is platinum or nickel which is present in an amount of 0.1 to 2% by weight based on the total weight of catalyst.

9. A process as claimed in any one of the preceding claims in which the alkylbenzene is a xylene or a mixture of xylenes containing from 0 to 25% by weight of ethylbenzene.

## Patentansprüche

1. Verfahren zur Isomerisation von Alkylbenzolen, bei welchem eine Beschickung aus einem Alkylbenzol oder aus einem Gemisch von Alkylbenzolen unter Isomerisationsbedingungen in der Gas- oder Flüssigphase mit einem Katalysator in Berührung gebracht wird, dadurch gekennzeichnet, daß der Katalysator Zeolith Nu-4 enthält.

2. Verfahren nach Anspruch 1, bei welchem der Katalysator Zeolith Nu-4 in Form eines physikalischen Gemischs mit einem Verdünnungsmittel und/oder einem Bindermaterial enthält.

3. Verfahren nach Anspruch 2, bei welchem das Verdünnungsmittel aus Aluminiumoxid besteht, welches in einer Menge von 5 bis 95 Gew.-%, bezogen auf das Gesamtgewicht des Katalysators, vorliegt.

4. Verfahren nach einem der vorhergehenden Ansprüche, welches in der Gasphase bei einer Temperatur im Bereich von 100 bis 600°C und bei einem Druck im Bereich von 0,5 bis 50,7 bar (0,5 bis 50 at absolut) ausgeführt wird.

5. Verfahren nach einem der Ansprüche 1 bis 3, welches in der Flüssigphase bei einer Temperatur in Bereich von 0 bis 350°C und bei einem Druck im Bereich von 1 bis 202 bar (1 bis 200 at absolut) ausgeführt wird.

6. Verfahren nach einem der vorhergehenden Ansprüche, welches in Gegenwart von Wasserstoff ausgeführt wird, wobei der Wasserstoff in einer solchen Menge vorliegt, daß das Mol-Verhältnis Wasserstoff zu Alkylbenzol im Bereich von 1:1 bis 30:1 liegt.

7. Verfahren nach Anspruch 6, bei welchem der Katalysator neben Zeolith Nu-4 ein Metall der Gruppe VIII des Periodensystems enthält.

8. Verfahren nach Anspruch 7, bei welchem das Metall der Gruppe VIII aus Platin oder Nickel besteht, welches in einer Menge von 0,1 bis 2 Gew.-%, bezogen auf das Gesamtgewicht des Katalysators, vorliegt.

9. Verfahren nach einem der vorhergehenden Ansprüche, bei welchem das Alkylbenzol aus einem Xylol oder aus einem Gemisch von Xylolen, das 0 bis 25 Gew.-% Äthylbenzol enthält, besteht.

**Revendications**

1. Procédé pour l'isomérisation d'alkylbenzènes, qui comprend la mise en contact d'une alimentation d'un alkylbenzène ou d'un mélange d'alkylbenzènes, dans des conditions d'isomérisation en phase vapeur ou liquide, avec un catalyseur, caractérisé en ce que le catalyseur comprend de la zéolite Nu4.

2. Procédé suivant la revendication 1, dans lequel le catalyseur comprend de la zéolite Nu4 sous la forme d'un mélange physique avec un diluant et/ou un liant.

3. Procédé suivant la revendication 2, dans lequel le diluant est l'alumine qui est présente en une quantité de 5 à 95% en poids, sur base du poids total du catalyseur.

4. Procédé suivant l'une quelconque des revendications précédentes, dans lequel l'opération est exécutée en phase vapeur à une température de l'intervalle de 100 à 600°C et sous une pression de l'intervalle de 0,5 à 50,7 bars (0,5 à 50 atmosphères absolues).

5. Procédé suivant l'une quelconque des revendications 1 à 3, dans lequel l'opération est exécutée en phase liquide à une température de l'intervalle de 0 à 350°C et sous une pression de l'intervalle de 1 à 202 bars (1 à 200 atmosphères absolues).

6. Procédé suivant l'une quelconque des revendications précédentes, dans lequel l'opération est exécutée en présence d'hydrogène, l'hydrogène étant présent en une quantité telle que le rapport molaire de l'hydrogène à l'alkylbenzène soit situé dans l'intervalle de 1:1 à 30:1.

7. Procédé suivant la revendication 6, dans lequel le catalyseur comprend un métal du groupe VIII du tableau périodique conjointement avec la zéolite Nu4.

8. Procédé suivant la revendication 7, dans lequel le métal du groupe VIII est le platine ou le nickel, qui est présent en une quantité de 0,1 à 2% en poids, sur base du poids total du catalyseur.

9. Procédé suivant l'une quelconque des revendications précédentes, dans lequel l'alkylbenzène est un xylène ou un mélange de xylènes contenant 0 à 25% en poids d'éthylbenzène.